Europäisches Patentamt

(19) European Patent Office

Office européen des brevets

(11) Publication number: **0 277 370 B1**

(12) **EUROPEAN PATENT SPECIFICATION**

(45) Date of publication of patent specification: **03.06.92** (51) Int. Cl.⁵: **A61M 29/02**, A61M 25/00

(21) Application number: **87119371.0**

(22) Date of filing: **30.12.87**

(54) **Tandem balloon dilatation catheter.**

(30) Priority: **06.01.87 US 696**

(43) Date of publication of application:
**10.08.88 Bulletin 88/32**

(45) Publication of the grant of the patent:
**03.06.92 Bulletin 92/23**

(84) Designated Contracting States:
**CH DE FR GB IT LI NL**

(56) References cited:
**EP-A- 0 213 752
EP-A- 0 241 038
CH-A- 616 337
CH-A- 654 214
US-A- 4 323 071**

(73) Proprietor: **ADVANCED CARDIOVASCULAR
SYSTEMS, INC.
1395 Charleston Road
Mountain View, CA 94039-7101(US)**

(72) Inventor: **Gaiser, John W.
255 S. Rengstoroff No. 147
Mountain View, California 94040(US)**
Inventor: **Samson, Wilfred J.
19691 Farwell Avenue
Saratoga, California(US)**

(74) Representative: **Baillie, Iain Cameron et al
c/o Ladas & Parry, Altheimer Eck 2
W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

This invention relates to balloon dilatation catheters and more particularly to tandem balloon dilatation catheters.

### BACKGROUND

Balloon dilatation catheters have heretofore been provided. In the past when it has been necessary to utilize two different size balloons in connection with opening a stenosis in a vessel, it has been necessary to first insert the dilatation catheter with a smaller balloon and thereafter withdraw this dilatation catheter from the vessel and then insert a dilatation catheter having a balloon of larger diameter. Such a procedure in the past has been time consuming. There is therefore a need for a new and improved balloon dilatation catheter.

Swiss Patent No. 654,214 discloses a catheter of interest. This catheter is comprised of two balloons and a single flexible member associated therewith. Extending through the two balloons is a guide wire.

U.S. Patent No. 4,323,071 is another catheter of interest disclosing only one balloon with first and second tubular members the one encasing the other.

In general, it is an aspect of the present invention to provide a balloon dilatation catheter and method which utilizes tandem balloons to eliminate the need for catheter exchanges.

Another aspect of the invention is to provide a catheter and method of the above character in which the tandem balloons are of different sizes.

Another aspect of the invention is to provide a catheter and method of the above character which utilizes a multi-port adapter to permit independent rapid inflation and deflation of the tandem balloons.

Another aspect of the invention is to provide a dilatation catheter and method of the above character which is adapted to be used with movable and stationary guide wires.

Another aspect of the invention is to provide a dilatation catheter and method of the above character in which the balloons can be readily vented.

Additional aspects and features of the invention will appear from the following description in which the preferred embodiments are set forth in detail in conjunction with the accompanying drawings.

### SUMMARY OF THE INVENTION

Disclosed herein is a steerable dilatation catheter for angioplasty procedures comprising:

a first elongated tubular member;

a first dilatation balloon on the distal portion of the first tubular member;

a second dilatation balloon distal to the first balloon;

a guide wire or guiding member disposed within the catheter;

an adapter assembly provided on the proximal end of the catheter to facilitate inflation of the balloons, said catheter being characterized by:

a second elongated tubular member having an inner lumen extending therethrough which is disposed within the first elongated tubular member and which defines therebetween an annular lumen in fluid communication with the interior of the first dilatation balloon, a distal portion of the second tubular member extending through the interior of the first dilatation balloon being sealed about the distal portion of the second tubular member extending therethrough;

the first dilatation balloon being on the distal portion of the second tubular member, the inner lumen of the second tubular member being in fluid communication with the interior of the second dilatation balloon;

the guide wire being disposed within the inner lumen of the second tubular member which is fixed to prevent the removal thereof and which has a solid portion extending through the interior of the second dilatation balloon and out the distal end thereof with the distal end of the second balloon being sealed about the solid portion of the guiding means extending therethrough;

means provided on the proximal end of the guide wire or guiding member to torque the guiding means about the longitudinal axis thereof; and

the adapter assembly being provided on the proximal ends of the first and second tubular members to facilitate the independent inflation and deflation of the first dilatation balloon through the annular lumen and the independent inflation and deflation of the second dilatation balloon through the inner lumen of the second tubular member.

### THE DRAWINGS

Figure 1 is a side elevational view of a balloon dilatation catheter having tandem balloons incorporating the present invention.

Figure 2 is an enlarged cross-sectional view of the distal extremity of the catheter shown in Figure 1.

Figure 3 is a cross-sectional view taken along the line 3-3 of Figure 2.

Figure 4 is a cross-sectional view taken along the line 4-4 of Figure 2.

Figure 5 is a cross-sectional view of a balloon dilatation catheter incorporating the present invention similar to Figure 2, but showing the use of a movable guide wire.

Figure 6 is a cross-sectional view taken along the line 6-6 of Figure 5.

Figure 7 is a cross-sectional view taken along the line 7-7 of Figure 5.

Figure 8 is a cross-sectional view taken along the line 8-8 of Figure 5.

## DETAILED DESCRIPTION

In general, the tandem balloon dilatation catheter assembly of the present invention is comprised of an elongate member having first and second flow passages therein and having proximal and distal ends. First and second ballons are carried by the distal extremity of the flexible elongate member. The first balloon has a diameter substantially less than the diameter of the second balloon. A first balloon is disposed distally of the second balloon. Means is provided for establishing communication between the first of the flow passages and the interior of the first balloon as well as for establishing communication between the second lumen and the interior of the second balloon. Means is secured to the proximal extremity of the flexible elongate member permitting independent inflation and deflation of the first and second balloons through the first and second passages. Means is provided for venting at least one of the balloons.

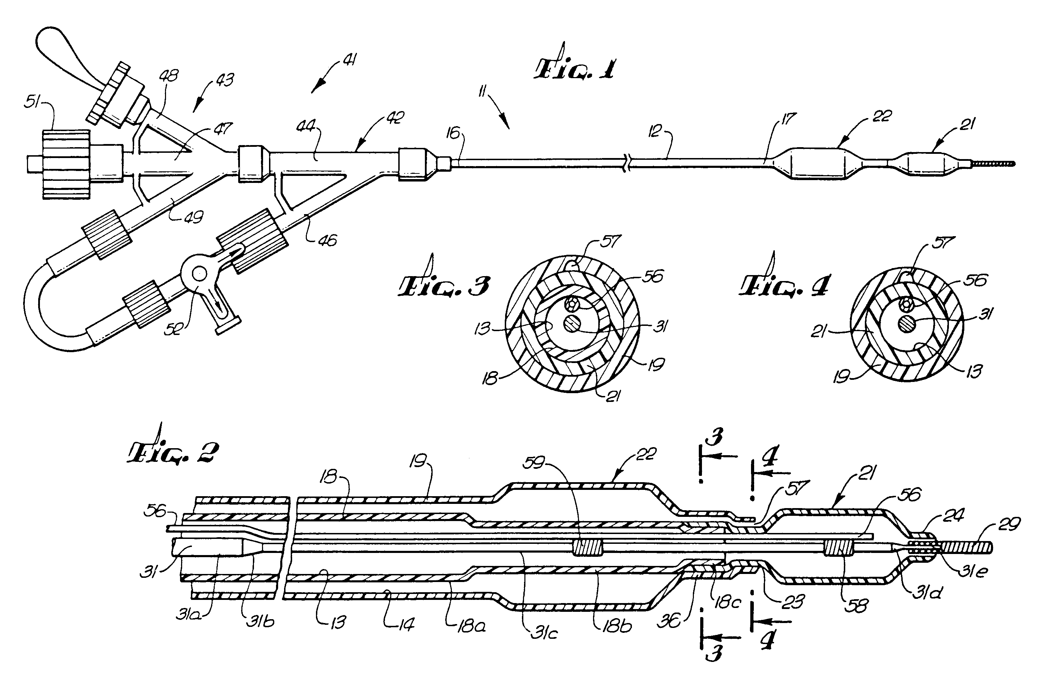

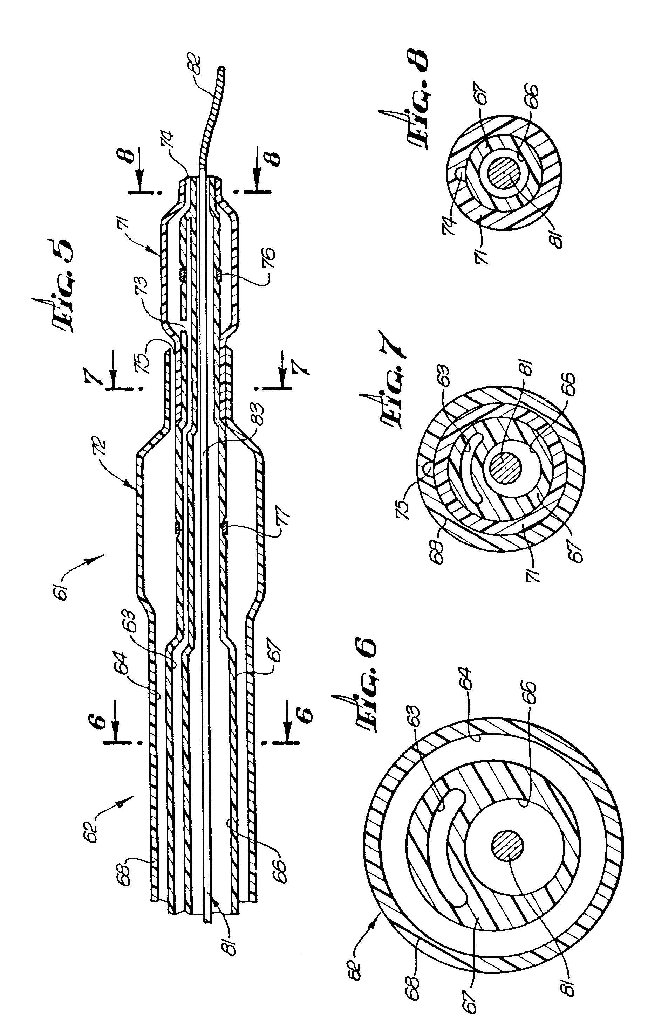

More specifically as shown in Figure 1, the tandem balloon dilatation catheter assembly 11 consists of a flexible elongate tubular member 12 formed of a suitable flexible material such as plastic. Suitable materials are disclosed in U.S. Letters Patent 4,323,071. The flexible elongate member 12 is formed so that it provides at least first and second flow passages 13 and 14 extending longitudinally thereof (see Figure 2). The flexible elongate member 12 has proximal and distal extremities 16 and 17. The flow passage 13 and 14 can be formed in a suitable manner such as by providing a co-axial construction for the tubular member 12 such as shown in Figure 2 and as disclosed in U.S. Letters Patent No. 4,323,071 in which an inner tubular member 18 is provided which has the flow passage 13 extending therethrough and an outer tubular member 19 is provided with the flow passage 14 being in the form of an annular flow passage extending between the exterior of the inner tubular member 18 and the interior of the outer tubular member 19. It should be appreciated that if desired, the flow passages 13 and 14 can be formed in a single tubular member by providing two flow passages or lumens 13 and 14 in the tubular member by suitable means such as by extrusion.

First and second tandem balloons 21 and 22 are carried by the first distal extremity of the flexible elongate member 12. The first balloon 21 is distal of the second balloon 22 and preferably has the smaller size or smaller outside diameter than the diameter of the second balloon 22. The first balloon 21 can be referred to as a distal balloon with the second balloon 22 being referred to as the proximal balloon.

As can be seen from Figure 2, means is provided for establishing communication between the first flow passage 13 and the interior of the balloon 21 by having the flow passage 13 open directly into the balloon 21. Means is also provided for establishing communication between the second flow passage 14 and the interior of the second balloon 22 by having the flow passage 14 open directly into the balloon 22. The inner tubular member 18 is provided with a continuous diameter extremity to near the distal extremity. The inner tubular member 18 is provided with necked-down portions 18b and 18c on its distal extremity of progressively smaller diameters. Portion 18b extends through the balloon 22 whereas portion 18c terminates at the junction of balloon 22 and the balloon 21.

The first or distal balloon 21 can be formed of a separate tubular balloon member or it can be formed integral in the distal extremity of the inner tubular member 18. As shown in Figure 2 it is formed as a separate tubular member or component which has its proximal necked-down extremity 23 secured to the portion 18c of the distal extremity of the inner tubular member 18 by suitable means such as by using heat shrinkable irradiated material for the balloon member or by utilizing an adhesive to form a fluid-tight seal. The necked-down distal extremity 24 of the balloon member 21 can be necked-down onto a spring tip 29 of a core wire 31 or by utilizing an adhesive to form suitable means such as a fluid-tight seal.

The spring tip 29 can be formed in a manner described in U.S. Patent No. 4,538,622 and is formed of a suitable radiopaque material such as tungsten or platinum or a combination of the two.

The spring tip 29 is secured to the distal extremity of the core wire 31 in a suitable manner such as by soldering and brazing. The core wire 31 can be formed of a suitable material such as stainless steel. Typically, it can be a diameter of 0.35 - 0.45 mm (.014 to .018 inch) with progressive tapers to smaller diameters along its distal extremity. As shown in Figure 2, the core wire 31 extends through the inner or first flow passage 13. The core wire 31 can have a portion 31a of a continuous diameter of 0.4 mm (.016 inch) from its proximal end for a distance of approximately 150 centimeters followed by a tapered portion 31b approximately 3 centimeters in length. The cylindrical portion 31c follows having a continuous diameter of

.008 inch followed by a tapered portion 31d three centimeters in length which is followed by a flattened portion 31e having a thickness of 0.05 -0.075 mm (.002 to .003 inch) and a length of .5 to 2 centimeters. The portion 31c extends through the balloons 21 and 22.

The second or proximal balloon 22 can be formed integral with the outer tubular member 19 or if desired, it also can be formed as a separate tubular member and bonded thereto by suitable means such as an adhesive.

If it is formed integral with the tubular member it can be formed of a heat shrinkable irradiated material in which the distal necked-down extremity 36 can be secured to the proximal extremity of the first balloon 21 by suitable means such as by a shrink fit or by the use of an adhesive.

An adapter assembly 41 is secured to the proximal extremity of the elongate member 12 and consists of a two-arm adapter 42 and a three-arm adapter 43. The two-arm adapter 42 consists of a central arm 44 and a side arm 46 and the three-arm adapter consists of a central arm 47 and side arms 48 and 49. The core wire or guide wire 31 extends through central arms 44 and 47 and is connected to a rotation limiting device 51. The arms 49 and 46 are in communication with the flow passages 13 and 14 respectively so that radiographic contrast liquid can be introduced into the passage for inflating and deflating the balloons 21 and 22. This can be accomplished by the use of an inflation/deflation device of the type disclosed by U.S. Patent No. 4,439,185 coupled to a valve fitting 52 connected to the arms 46 and 49.

Suitable means is provided for venting air from the first and second balloons 21 and 22 as they are filled with the radiographic contrast liquid. By way of example, a vent tube 56 of the type described in U.S. Letters Patent 4,323,071 can be utilized and introduced through the side arm 48 and into the first flow passage or lumen 13 until it extends near the distal extremity of the balloon 21. As can be seen, as the balloon is filled with radiopaque liquid, air in the balloon will be forced into the vent tube and vented to the atmosphere through its proximal extremity. Similarly, another vent tube (not shown) can be provided which is inserted into the second lumen or flow passage 14 and extended into the distal extremity of the balloon 22 so that any air collected in that balloon can also be vented to the atmosphere through the proximal extremity of the vent tube. It should be appreciated that alternative venting means can be utilized, such as a self-venting means in which a small passage 57 is provided (see Figures 3 and 4) for venting the proximal balloon 22 to the atmosphere. The above alternative vent design may also be used on the distal balloon 21, replacing the vent tube 56.

One or more markers which are substantially radiopaque, such as markers 58 and 59 can be provided in each of the balloons 21 and 22 so that the general location of the balloons 21 and 22 can be viewed under a fluoroscope. It should be appreciated that even though the markers 58 and 59 have been shown intermediate the ends of each of the balloons, that if desired two markers can be provided in each of the balloons with one near the distal extremity of the balloon and the other near the proximal extremity of the balloon so that the physician utilizing the catheter can readily ascertain the position of the balloons of the catheter with respect to a stenosis.

Operation and use of the tandem balloon catheter assembly shown in Figures 1 and 2 may now be briefly described as follows in performing the method of the present invention. In utlizing the dilatation catheter assembly, a guiding catheter (not shown) is first inserted into the vessel by the use of a guide wire (not shown). The guide wire is then removed. The dilatation catheter assembly 11 prior to introduction of the guiding catheter has been prepared for insertion into the guiding catheter. This has been done by inflating the balloons 21 and 22 by inserting a suitable liquid such as a radiopaque contrast medium into the arms 46 and 49 to inflate the balloons 21 and 22 to ensure that all air is removed from the balloons. The balloons are then deflated by removing the radiopaque liquid.

Assuming that the tandem balloon dilatation catheter assembly is of a type shown in Figure 1 which uses a guidewire which is fixed at its distal extremity, the dilatation catheter is inserted into the vessel by introducing the same into the guiding catheter and advancing the spring tip across the stenosis. The smaller or distal ballon 21 is advanced across the stenosis and inflated by introducing radiopaque contrast liquid into the balloon 21 through the arm 49. Thereafter to obtain additional enlargement of the opening in the stenosis, the second balloon 22 can then be advanced into the opening formed by the first balloon 21. It is then inflated by introducing radiopaque liquid through the arm 46. After this balloon 22 has been inflated, it is deflated and the catheter assembly can then be removed. Assuming that the angioplasty procedure has been successful, the guiding catheter can thereafter be removed.

Another embodiment of the invention is shown in Figures 5-8 in which a tandem balloon dilatation catheter assembly 61 is shown. The assembly 61 consists of an elongate flexible member 62 which is provided with proximal and distal ends. It also has at least first and second flow passages or lumens 63 and 64 which extend longitudinally of the elongate flexible member 62. In addition, in the embodiment of the invention shown in Figure 5, an additional or third flow passage or lumen 66 is provided in the elongate flexible member 62. The flow passages or lumens 63, 64 and 66 can be provided in any suitable manner. For example, as shown in Figure 6, a partial co-axial type construction can be utilized in which the elongate flexible member 62 is comprised of an inner tubular member 67 and an outer tubular member 68. The inner tubular member 67 has a flow passage or lumen 63 extending therethrough which serves as a balloon inflation lumen. The lumen 66 may also be utilized for distal radiopaque dye injection. The second flow passage or lumen 64 is formed by the annular space provided between the inner and outer tubular members 67 and 68. The third lumen 66 is provided within the inner tubular member 67 as shown particularly in Figure 6. The lumen 66 serves as the guide wire lumen and the lumen 63 serves as the inflation/deflation lumen. The inflation/deflation lumen 63 as shown can be substantially moon-shaped in cross section whereas the guide wire lumen 66 is generally circular in cross section. In this manner, it is possible to provide two lumens in the same inner tubular member without substantially increasing the overall cross-sectional diameter of the inner tubular member 67.

It should be appreciated that if desired, all of the three flow passages or lumens 63, 64 and 66 can be formed in a single tubular member by providing two inflation lumens such as the inflation lumen 63 within the same tubular member and by providing a centrally disposed guide wire lumen 66 in the same tubular member. This can be readily accomplished by extruding such an elongate flexible member. The inner and outer tubular member 67 and 68 can be formed of a suitable material such as plastic, as hereinbefore described.

First and second or distal and proximal dilatation balloons 71 and 72 are carried by the distal extremity of the elongate flexible member 62 and are arranged in tandem or in series as disclosed with respect to the first embodiment of the present invention. As hereinbefore described, integral or separate balloons can be provided. Thus as shown in Figure 5, a separate balloon 71 has been provided in which distal extremity of the ballon is necked down and sealed to the distal extremity of the inner tubular member 67 by suitable means such as an adhesive or a shrink fit. Similarly, the proximal extremity of the first balloon 71 is necked down and also fitted over the inner tubular member 67 and secured thereto by suitable means such as an adhesive provide a fluid-tight seal.

Means is provided for establishing communication between the balloon inflation lumen 63 and the interior of the first balloon 71 and consists of a port 73 formed in the side wall forming the inner tubular member 67 adjacent the proximal extremity of the balloon 71 which opens into the balloon inflation lumen 63. Means is provided for venting the balloon 71 so that entrapped air therein can escape when the balloon is inflated and consists of the previously mentioned self-venting means. As described, the self-vent is formed as a small bore or passage 74 in the distal extremity of the balloon 71. Alternatively, a vent tube or port leading into an inner lumen 66 can be used.

The second balloon 72 as shown is formed integral with the outer tubular member 68 and has its distal extremity secured over the proximal extremity of the first balloon 71 and is secured thereto by suitable means such as an adhesive to also provide a fluid-tight seal. As shown, the interior of the second balloon 72 is in communication with the balloon inflation lumen 64. Means is also provided for venting the balloon 72. This also can be a self-vent provided by a small passage or bore 75 in the distal extremity of the balloon. Alternatively, a vent tube can be used.

Radiopaque markers 76 and 77 of a suitable type, as for example, gold bands are mounted within the first and second balloons 71 and 72 so that the positioning of the balloons during an angioplasty procedure can be ascertained. As shown, a single marker 76 is provided between the distal and proximal extremities of the first balloon 71 and similarly, a single marker 77 has been provided between the proximal and distal extremities of the second balloon 72. It should be appreciated that if desired additional markers can be utilized in each of the balloons as, for example, placing a marker adjacent the distal and proximal extremities of the balloon rather than in the center of the balloon.

A guide wire 81 of a conventional construction is provided for use with the catheter assembly 61. It is preferably of the torquable type and is provided with a spring tip 82 which is secured to a shaft 83.

With the construction shown, to reduce diameters, the portions of the inner and outer tubular members 67 and 68 near the distal extremities have been necked down to accommodate the balloons 71 and 72 and to make it possible to provide relatively low profiles for the balloons while still retaining good flow characteristics in the distal extremities of the inner and outer tubular members 67 and 68. Thus there has been provided a catheter assembly which still provides good flow characteristics with relatively thick walls being provided to provide added stiffness for the catheter assembly. The embodiment of the catheter assembly 61 can be provided with an adapter assembly of the type similar to the adapter assembly described in connection with the catheter assembly shown in Figure 1 which is provided with balloon inflation ports and a guide wire port.

In connection with the present invention, tandem balloon catheter assemblies have been provided in which the first balloon has diameters ranging from 1.5 and 2.5 millimeters and the second or larger balloon has diameters ranging from 2.5 to 4.0 millimeters.

The procedure for use of the tandem balloon dilatation catheter assembly of the type shown in Figure 5 which utilizes a movable guide wire is very similar to that described in Figure 1 with the exception that the guide wire 81 is first introduced into the guiding catheter with the spring tip being advanced into the stenosis. The catheter assembly 61 is advanced over the guide wire. The balloons 71 and 72 are used in a similar two-step procedure to open the stenosis. After the two balloons have been inflated and deflated, the catheter assembly 61 with the guide wire 81 can be removed. Thereafter, the guiding catheter can be removed.

From the foregoing it can be seen that the balloon dilatation catheter assembly of the present invention is particularly useful in opening very tight stenoses by utilizing a two-step procedure. The smaller distal balloon can be utilized to enter the tight stenosis because of its smaller profile. After a partial opening of the tight stenosis with the smaller balloon, the higher profile proximal balloon can be advanced into the stenosis and inflated to complete the dilatation of the stenosis.

By utilizing such tandem balloon dilatation catheter assemblies, it is possible to eliminate the use of a plurality of balloon dilatation catheters of increasing size in an angioplasty procedure to achieve the desired opening of the stenosis. By eliminating the use of a plurality of catheters and by providing balloon dilatation catheter assemblies having tandem balloons, it is possible to reduce the amount of time and manipulations required in performing an angioplasty procedure.

It should be appreciated that although the tandem balloon dilatation catheter assembly shown in Figure 1 only utilizes two balloons, it is poossible if desired to provide additional balloons in tandem having still larger diameters if this is found to be necessary.

## Claims

1. A steerable dilatation catheter for angioplasty procedures comprising:

   a first elongated tubular member (19);

   a first dilatation balloon (22) on the distal portion of the first tubular member (19);

   a second dilatation balloon (21) distal to the first balloon (22);

   a guide wire or guiding member (31) disposed within the catheter;

   an adapter assembly (41) provided on the proximal end of the catheter to facilitate inflation of the balloons (21,22), said catheter being characterized by:

   a second elongated tubular member (18) having an inner lumen (13) extending therethrough which is disposed within the first elongated tubular member (19) and which defines therebetween an annular lumen (14) in fluid communication with the interior of the first dilatation balloon (22), a distal portion (18b) of the second tubular member (18) extending through the interior of the first dilatation balloon (22) being sealed about the distal portion of the second tubular member extending therethrough;

   the first dilatation balloon being on the distal portion of the second tubular member, the inner lumen (13) of the second tubular member being in fluid communication with the interior of the second dilatation balloon (21);

   the guide wire (31) being disposed within the inner lumen (13) of the second tubular member (18) which is fixed to prevent the removal thereof and which has a solid portion (31c) extending through the interior of the second dilatation balloon and out the distal end thereof with the distal end of the second balloon being sealed about the solid portion of the guiding means extending therethrough;

   means (51) provided on the proximal end of the guide wire or guiding member to torque the guiding means (31) about the longitudinal axis thereof; and

   the adapter assembly (41) being provided on the proximal ends of the first (19) and second (18) tubular members to facilitate the independent inflation and deflation of the first dilatation balloon through the annular lumen and the independent inflation and deflation of the second dilatation balloon through the inner lumen of the second tubular member.

2. The assembly as claimed in Claim 1 further characterized in that said guiding means (31) is carried by the distal extremity of said catheter.

3. The assembly as claimed in Claim 2 further characterized in that said guiding means (31) is secured to the distal extremity of said catheter.

4. An assembly as claimed in Claim 2 further characterized in that said catheter has a third lumen passage (66) extending therethrough and wherein said guiding means (83) is removably mounted in said third lumen (66).

5. An assembly as claimed in any one of Claims 1-4 together with means for venting (56) entrapped air in said first (22) and second (21) balloons.

**Revendications**

1. Cathéter dirigeable de dilatation pour opérations d'angioplastie, comprenant :

un premier organe tubulaire allongé (19),

un premier ballon (22) de dilatation placé sur la partie distale du premier organe tubulaire (19),

un second ballon (21) de dilatation ayant une position distale par rapport au premier ballon (22),

un organe ou fil de guidage (31) placé dans le cathéter,

un ensemble adaptateur (41) placé à l'extrémité proximale du cathéter et destiné à faciliter le gonflage des ballons (21, 22), le cathéter étant caractérisé par

un second organe tubulaire allongé (18) ayant une lumière interne (13) qui le traverse, disposé à l'intérieur du premier organe tubulaire allongé (19) et qui délimite avec lui une lumière annulaire (14) communiquant avec l'intérieur du premier ballon de dilatation (22), une partie distale (18b) du second organe tubulaire (18) passant à l'intérieur du premier ballon de dilatation (22) étant en coopération étanche autour de la partie distale du second organe tubulaire passant à l'intérieur,

le premier ballon de dilatation se trouvant sur la partie distale du second organe tubulaire, la lumière interne (13) du second organe tubulaire communiquant avec l'intérieur du second ballon de dilatation (21),

le fil de guidage (31) étant disposé dans la lumière interne (13) du second organe tubulaire (18), qui est fixé afin qu'il ne puisse pas être extrait et qui a une partie pleine (31c) passant à l'intérieur du second ballon de dilatation et sortant par son extrémité distale, l'extrémité distale du second ballon coopérant de façon étanche autour de la partie pleine du dispositif de guidage qui passe à l'intérieur,

un dispositif (51) étant placé à l'extrémité proximale du fil ou organe de guidage afin qu'il applique un couple au dispositif de guidage (31) autour de son axe longitudinal, et

un ensemble adaptateur (41) placé aux extrémités proximales du premier (19) et du second (18) organe tubulaire afin que le gonflage et le dégonflage indépendants du premier ballon de dilatation par la lumière annulaire et le gonflage et le dégonflage indépendants du second ballon de dilatation par la lumière interne du second organe tubulaire soient facilités.

2. Ensemble selon la revendication 1, caractérisé en outre en ce que le dispositif de guidage (31) est porté par l'extrémité distale du cathéter.

3. Ensemble selon la revendication 2, caractérisé en outre en ce que le dispositif de guidage (31) est fixé à l'extrémité distale du cathéter.

4. Ensemble selon la revendication 2, caractérisé en outre en ce que le cathéter a un troisième passage (66) d'une lumière formée à l'intérieur, et dans lequel le dispositif de guidage (83) est monté de façon amovible dans la troisième lumière (66).

5. Ensemble selon la revendication 1,2,3, ou 4, comportant un dispositif de ventilation (56) de l'air piégé dans le premier ballon (22) et dans le second ballon (21).

**Patentansprüche**

1. Lenkbarer aufdehnbarer Katheter für Angioplastikmaßnahmen mit

einem ersten langgestreckten rohrförmigen Glied (19);

einer auf dem distalen Teil des ersten rohrförmigen Gliedes (19) angeordneten, ersten Aufdehnballon (22);

einem distal von dem ersten Ballon (32) angeordneten, zweiten Aufdehnballon (21);

einem in dem Katheter angeordneten Führungsdraht oder Führungsglied (31);

einer an dem proximalen Ende des Katheters angeordneten Zwischenstückanordnung (41) zum Erleichtern des Füllene des Ballons (21, 22), wobei der Katheter Gekennzeichnet ist durch:

ein zweites langgestrecktes rohrförmiges Glied (18), das von einem Innenlumen (13) durchsetzt ist und das in dem ersten langgestreckten rohrförmigen Glied (19) angeordnet ist und mit ihm ein ringförmiges Lumen (14) begrenzt, das mit dem Innern des ersten Aufdehnballons (22) in Strümungsverbindung steht, wobei das erste rohrförmige Glied einen distalen Teil (18b) besitzt, der das Innere des ersten Aufdehnballons (22) durchsetzt, das an dem distalen Teil des dieses Innere durchsetzenden zweiten rohrförmigen Gliedes abgedichtet ist;

der erste Aufdehnballon an dem distalen Teil des zweiten rohrförmigen Gliedes vorgesehen ist, dessen Innenlumen (13) mit dem Innern des zweiten Aufdehnballons (21) in Strömungsverbindung steht;

der Führungsdraht (31) in dem Innenlumen (13) den zweiten rohrförmigen Gliedes (18) angeordnet ist, das gegen seine Abnahme fixiert ist und einen massiven Teil (31c) besitzt, der sich durch das Innere des zweiten Aufdehnballons hindurch und aus dessen distalem Ende heraus erstreckt, und das distale Ende des zweiten Ballons um den massiven Teil des sich durch dieses distale Ende erstreckenden Führungsmittels herum abgedichtet ist:

an dem proximalen Ende des Führungsdrahtes oder Führungsgliedes eine Einrichtung (51) zum Drehmomentbelasten des Führungsmittels (31) um seine Längsachse vorgesehen ist; und

die Zwischenstückanordnung (41) an den proximalen Enden des ersten (19) und zweiten (18) rohrförmigen Gliedes vorgesehen ist, um die das unabhängige Füllen und Entleeren des ersten Aufdehnballons durch das ringförmige Lumen unt das unabhängige Füllen und Entleeren des zweiten Aufdehnballons durch das Innenlumen des zweiten rohrförmigen Gliedes zu erleichtern.

2. Anordnung nach Anspruch 1, dadurch gekennzeichnet, daß der Katheter das Führungsmittel (31) an seinem distalen Ende trägt.

3. Anordnung nach Anspruch 2, dadurch gekennzeichnet, daß das Rührungsmittel (31) an dem distalen Ende des Katheters befestigt ist.

4. Anordnung nach Anspruch 2, dadurch gekennzeichnet, daß der Katheter von einem Kanal durchsetzt ist, der ein drittes Lumen (66) bildet, in dem das Führungsmittel (83) abnehmbar montiert ist.

5. Anordnung nach Anspruch 1,2,3 oder 4 mit einer Entlüftungseinrichtung (56) für den Abzug von in dem ersten (22) und dem zweiten (21) Ballon eingeschlossener Luft.

Fig. 1

Fig. 2

Fig. 3

Fig. 4

_Fig. 5_

_Fig. 6_

_Fig. 7_

_Fig. 8_

EP 0 277 370 B1